# EUROPEAN PATENT APPLICATION

(11) **EP 4 736 924 A1**
(43) Date of publication of application: **06.05.2026**
(21) Application number: 25213606.4
(22) Date of filing: 05.11.2025
(51) Int. Cl.: A61M 21/00, A61N 5/06, B60Q 3/47, H05B 35/00

(54) **A TRAIN CABIN SYSTEM FOR FATIGUE MANAGEMENT**

(30) Priority: 05.11.2024 NL 2038995
(71) Applicant: Dual Inventive Holding B.V., 5061 KG Oisterwijk (NL)
(72) Inventor: VAN DER POEL, Lex Josephus Maria, 5061 KG Oisterwijk (NL)
(74) Representative: Algemeen Octrooi- en Merkenbureau B.V.

(57) **Abstract**

A train cabin lighting system for managing the fatigue of a train operator comprises a cabin interior configured to accommodate the operator during operation. The system includes a light-emitting apparatus arranged within the cabin, which comprises at least one light source capable of emitting light at different wavelengths. A control unit is operatively connected to the light-emitting apparatus, and it is configured to control the emission of light based on predefined protocols. The system is characterized by the light-emitting apparatus being configured to emit blue light at a wavelength between 460 nm and 480 nm. Additionally, an adaptive lighting control system dynamically adjusts one or more of the intensity, spectral composition, and emission angle of the light based on real-time feedback from environmental and operator-specific conditions, thereby managing the operator's fatigue.

## Description

### Technical field

The present invention relates to a train cabin system, specifically designed to manage operator fatigue by utilizing controlled light emission.

### Background

In modern railway operations, the role of the train operator is critical for the safe and timely transport of passengers and goods. Train operators are often required to maintain high levels of concentration and alertness over extended periods, particularly during night shifts or long-distance routes. While various systems are in place to assist operators, such as automated train control systems and external alert mechanisms, the issue of operator fatigue remains a significant challenge. Fatigue impairs cognitive function, slows reaction times, and increases the likelihood of human error, which can have serious consequences in high-risk environments such as rail transport.

Current fatigue management solutions in the railway industry primarily focus on scheduling adequate rest periods between shifts, implementing safety protocols, and utilizing in-cabin alarms to alert operators in critical situations. Additionally, some efforts have been made to introduce personal fatigue-management tools, such as wearable devices and fatigue-detection systems that monitor physiological signals like eye movement or head nodding. However, these systems are reactive rather than preventative, only alerting operators after fatigue has already impacted their performance. This approach does not address the underlying issue of maintaining consistent alertness, especially during prolonged night shifts or during periods of reduced natural light.

The limitations of these existing solutions are evident in their inability to actively influence an operator's alertness before signs of fatigue become dangerous. Alarms and alerts are often disruptive, startling operators instead of helping them maintain a steady level of concentration. Furthermore, wearable fatigue detection devices can be uncomfortable or intrusive, limiting their widespread adoption. Additionally, none of these solutions take into account the natural circadian rhythm of operators, which is a key factor in managing fatigue, particularly during night-time operations.

In recent years, advancements in lighting technology, specifically the development of LED-based systems, have enabled new possibilities for manipulating light spectra and intensities in ways that could enhance alertness and cognitive performance. Blue-enriched white light, generally characterized by color temperatures of 6300K or higher, has gained significant attention across various industries for its potential to improve alertness and reduce subjective sleepiness during night work. Scientific studies have demonstrated that exposure to blue-enriched light can lead to faster reaction times, improved alertness, and reduced fatigue, particularly in controlled environments such as laboratories and some field settings.

This has led to considerations for implementing blue-enriched lighting in train cabins as a countermeasure for managing fatigue among rail workers and train operators. However, the application of blue-enriched light in these settings presents several notable drawbacks. Firstly, exposure to high-intensity blue light, especially during night shifts, can interfere with the body's natural circadian rhythms by suppressing melatonin production. This disruption can result in long-term sleep issues and other health risks for workers who frequently operate during nighttime hours. Secondly, blue-enriched light has been linked to increased eye strain and visual discomfort, which could impair the visual acuity necessary for performing safety-critical tasks in railway operations.

A further concern is the impact of altered lighting conditions on the perception of essential visual and vital information, such as railway signs and signals, both within the cabin and along the track. Color perception under blue-enriched lighting can be distorted, making it more difficult to accurately distinguish critical visual cues. This could potentially lead to the misinterpretation of signals or delayed recognition of important information, thereby compromising safety. Additionally, studies have shown that while blue-enriched light can enhance subjective feelings of alertness, it does not always correspond to improved performance in precision tasks. In some cases, exposure to blue-enriched light has even been found to impair accuracy, which is particularly concerning in train operations that demand precise control and decision-making.

Moreover, the effectiveness of blue-enriched light can vary between individuals, presenting challenges in creating a standardized solution that benefits all workers equally. This variability further complicates the practical implementation of such lighting systems in train cabins, where consistency in performance and safety is paramount.

Thus, while the use of blue-enriched lighting has shown promise in certain contexts, its application in train cabins must address several critical issues, including minimizing the disruption of circadian rhythms, reducing the risk of eye strain, ensuring optimal color perception for safety-critical information, and avoiding any potential negative impact on task performance. There is a clear need for a lighting system in train cabins that enhances alertness and performance while overcoming the limitations associated with current blue-enriched lighting technologies.

The present invention aims to address these challenges by providing a lighting system for train cabins that improves the alertness and cognitive performance of rail workers and train operators without compromising safety or comfort. The present invention aims to optimize light intensity, spectral composition, and application timing, the system seeks to enhance worker alertness while maintaining the necessary visual clarity for distinguishing signals and other critical information. Furthermore, the invention seeks to minimize adverse effects on circadian rhythms and individual variability in response to the light, offering a more balanced and effective solution for fatigue management in railway operations.

### Summary

The above mentioned and other objects are achieved by the claimed invention.

One aspect of the present invention relates to a train cabin lighting system for fatigue management of a train operator.

A train cabin may be understood as the interior space within any type of locomotive, rail vehicle, or also specialized railway maintenance equipment, such as a ballast undercutter or "kettinghor." This space is designed to accommodate individuals responsible for operating or overseeing the machinery or train functions. In the context of standard locomotives, this cabin may house a machinist or train driver, whereas in specialized equipment like the kettinghor, the cabin is referred to as the operator's cabin or control cabin. This cabin is where machine operators, ballast undercutter operators, and railway maintenance technicians control the movement of the machinery and monitor processes, such as the cleaning and redistribution of ballast along railway tracks.

Fatigue management refers to systems or mechanisms designed to mitigate or prevent the negative effects of fatigue on the performance of individuals working within such cabins, whether these individuals are train drivers, machinists, or railway maintenance personnel. This is particularly relevant during prolonged work hours, night shifts, or when operating complex machinery like the kettinghor, where constant alertness is required to ensure safe and efficient railway operations. The system comprises a cabin interior configured to accommodate a train operator during operation. A cabin interior may be understood as the enclosed area in the train where the operator performs their duties, often designed ergonomically to ensure the operator's comfort and ease of access to control systems. This arrangement provides an environment where the operator can be exposed to controlled lighting that aims to manage fatigue and enhance alertness.

The system further comprises a light-emitting apparatus arranged within the cabin, said apparatus comprising at least one light source capable of emitting light in different wavelengths. A light-emitting apparatus may be understood as a device or assembly that generates and projects light, which can include technologies such as LEDs (Light Emitting Diodes). A light source capable of emitting light in different wavelengths refers to a system that can produce light across various parts of the light spectrum, such as visible light and particularly blue-enriched light. The ability to emit different wavelengths enables the system to provide specific types of light, such as blue light, which has been shown to positively influence alertness and circadian rhythms. An effect of this arrangement is that the operator can be exposed to specific wavelengths of light optimized for reducing fatigue and improving cognitive performance, particularly during night-time operations or extended shifts.

The system includes a control unit operatively connected to the light-emitting apparatus, configured to control the emission of light based on predefined protocols. A control unit may be understood as an electronic component or system that regulates the functions of the light-emitting apparatus, such as adjusting the intensity, color, and duration of light output. Predefined protocols refer to a set of programmed instructions that dictate the behavior of the lighting system according to specific operational conditions, such as the time of day or operator-specific settings. The effect of this arrangement is that the system can automatically manage the light output in a consistent and predictable manner, ensuring that the operator is exposed to the appropriate type and intensity of light for fatigue management without the need for manual intervention.

The system is further characterized by the light-emitting apparatus being configured to emit blue light at a wavelength between 460 nm and 480 nm. Blue light refers to the part of the visible light spectrum typically associated with wavelengths between approximately 400 nm and 500 nm, and the specific range of 460 nm to 480 nm has been identified as particularly effective in influencing alertness and suppressing melatonin production, thereby managing fatigue. The effect of this arrangement is that the light-emitting apparatus can emit blue light at the most effective wavelengths for enhancing cognitive function and maintaining wakefulness during long work periods, particularly in low-light conditions or night shifts.

Additionally, the system includes an adaptive lighting control system that dynamically adjusts one or more of the intensity, spectral composition, and emission angle of the light, based on real-time feedback from environmental and operator-specific conditions, to manage the operator's fatigue. An adaptive lighting control system refers to a system that modifies its behavior in response to changing conditions. Intensity refers to the brightness of the light, spectral composition refers to the mixture of wavelengths emitted, and emission angle refers to the direction or spread of the light. Real-time feedback may include data from environmental sensors, such as ambient light detectors, or physiological data from the operator, such as heart rate variability or eye movement. The effect of this arrangement is that the system can optimize lighting conditions dynamically, ensuring that the light is adjusted according to both the operator's physiological state and the environmental conditions. This allows the system to maintain optimal alertness for the operator throughout varying work conditions, reducing the risk of fatigue-related errors and improving overall safety and performance.

In an example, the train cabin lighting system adjusts the intensity of the light in a range from 50 to 1000 lux, more preferably from 200 to 600 lux, and even more preferably from 300 to 500 lux. It may be provided that light intensity refers to the brightness level of the emitted light, measured in lux. The effect of this feature is to ensure that the operator is exposed to an appropriate level of brightness that maximizes alertness while minimizing eye strain, especially under varying lighting conditions inside and outside the cabin.

In an example, the train cabin lighting system adjusts the intensity of the light based on real-time feedback from ambient light sensors. It may be provided that ambient light sensors detect external light levels and send data to the adaptive lighting control system. The effect of this feature is to enable the system to dynamically balance artificial and natural light, optimizing energy use and improving the operator's visual comfort by avoiding excessive or insufficient lighting during operation.

In an example, the adaptive lighting control system adjusts the spectral composition of the light between 435 nm and 500 nm, more preferably between 450 nm and 480 nm, and most preferably between 460 nm and 470 nm. It may be provided that spectral composition refers to the range of wavelengths emitted by the light source. The effect of this feature is to provide wavelengths that specifically influence the operator's circadian rhythm and cognitive alertness, particularly with blue light, which has been shown to suppress melatonin and improve alertness.

In an example, the light-emitting apparatus is configured to emit blue-enriched light with a correlated color temperature (CCT) of 6300K or higher. It may be provided that CCT describes the color characteristics of the light, where higher values correspond to cooler, blue-enriched light. The effect of this feature is that the operator is exposed to blue-enriched light, which has been demonstrated to enhance alertness and improve cognitive function during periods of low natural light or night shifts.

In an example, the adaptive lighting control system dynamically adjusts the emission angle of the light, more preferably between 20 and 90 degrees, and most preferably between 30 and 60 degrees. It may be provided that the emission angle refers to the angle at which light is directed from the source. The effect of this feature is to ensure that light is targeted appropriately within the cabin, optimizing the operator's exposure to fatigue-reducing light without causing discomfort or glare.

In an example, the emission angle is adjusted based on the operator's seating position detected through a sensor. It may be provided that sensors track the operator's seating posture and location, allowing the system to adjust the direction of the light accordingly. The effect of this feature is that the operator receives light in the most effective position for fatigue management, ensuring consistency in exposure even as the operator changes positions.

In an example, the train cabin lighting system further comprises a feedback system that monitors operator-specific physiological conditions, including heart rate variability, blink rate, or eye movement, to control the emission of light in real time. It may be provided that physiological conditions are tracked via sensors or wearable devices, providing data that reflects the operator's state of fatigue. The effect of this feature is that the system can personalize lighting conditions, making real-time adjustments to prevent fatigue from reaching dangerous levels.

In an example, the physiological feedback is used to adjust the duration and intensity of blue light exposure. It may be provided that adjustments in duration and intensity are based on the operator's current state, as indicated by physiological metrics such as heart rate or blink rate. The effect of this feature is to create a customized lighting environment that maximizes alertness while preventing overexposure to blue light, which could otherwise disturb circadian rhythms.

In an example, the adaptive lighting control system adjusts the duration of blue light emission in cycles, with each cycle lasting between 10 and 60 minutes, more preferably between 15 and 45 minutes, and most preferably between 20 and 30 minutes. It may be provided that light is emitted in timed intervals, allowing for breaks between cycles. The effect of this feature is that the system can provide periodic stimulation to maintain alertness without causing overexposure, thus helping to preserve the operator's circadian balance.

In an example, the lighting system provides intermittent blue light exposure interspersed with periods of natural light to minimize circadian disruption. It may be provided that the system alternates between artificial and natural light sources. The effect of this feature is to simulate more natural lighting conditions, reducing the likelihood of disrupting the operator's sleep-wake cycle while still maintaining necessary alertness during work hours.

In an example, the train cabin lighting system further comprises a predefined illumination protocol that includes one or more of a predefined duration, pulse shape, duty cycle, intensity, wavelength, or lux of the illuminated light. It may be provided that an illumination protocol refers to a set of predetermined parameters that govern how light is emitted during operation. The effect of this feature is to provide a structured and repeatable method for optimizing lighting conditions to reduce fatigue, ensuring consistent results across various operating conditions.

In an example, the illumination protocol adjusts automatically based on the operator's shift schedule, providing more intense blue light during early night shifts and reducing exposure toward the end of the shift. It may be provided that the system takes into account the operator's work schedule and adapts the light output accordingly. The effect of this feature is to provide dynamic support for the operator's alertness, gradually easing the transition from work to rest by reducing blue light exposure toward the end of a shift.

In an example, the light-emitting apparatus is configured to emit photons at a wavelength between 435 nm and 500 nm, more preferably between 450 nm and 480 nm, and most preferably between 460 nm and 470 nm. It may be provided that the wavelength refers to the specific part of the light spectrum emitted. The effect of this feature is to ensure that the most effective wavelengths for alertness and fatigue management are consistently emitted, enhancing the operator's cognitive performance.

In an example, the light-emitting apparatus is arranged for diffusion of light onto a respective eye of the rail worker, ensuring even distribution of light across the operator's visual field. It may be provided that light diffusion refers to the way light spreads out from its source to cover a larger area. The effect of this feature is to avoid creating hotspots of light, which could cause glare or discomfort, while ensuring that both eyes receive uniform exposure to fatigue-reducing light.

In an example, the light-emitting apparatus is positioned such that it irradiates light from above, allowing light to reach the bottom of the retina to maximize circadian influence. It may be provided that irradiating from above targets the lower retina, which has been shown to have a significant influence on non-visual light responses. The effect of this feature is to maximize the biological impact of the light on the operator's circadian system, improving alertness and cognitive performance.

In an example, the emission angle is optimized to target the lower retina, stimulating non-visual photoreceptors responsible for alertness. It may be provided that the angle is adjusted to ensure the light reaches photoreceptors in the retina responsible for circadian regulation. The effect of this feature is that the operator's alertness is enhanced through targeted light exposure, which directly influences biological processes related to fatigue and wakefulness.

In an example, the train cabin lighting system further comprises a gateway or server that links the lighting system to a remote control system for adaptive control of settings such as intensity, spectral composition, emission angle, or personalized operator settings. It may be provided that the gateway or server facilitates communication between the lighting system and a remote device or network. The effect of this feature is that it allows the lighting system to be controlled and adjusted remotely, ensuring that changes in lighting conditions can be made without the need for manual intervention within the cabin, providing real-time adaptability even from a distance.

In an example, the remote system allows for operator-specific customization, adjusting the light output based on individual needs for intensity, wavelength, or duration. It may be provided that operator-specific customization refers to the ability of the remote system to modify lighting conditions according to the specific preferences or physiological needs of the operator. The effect of this feature is to create a more personalized lighting environment that adapts to the unique requirements of each operator, enhancing comfort and effectiveness in managing fatigue.

In an example, the adaptive lighting control system is configured to be integrated into the existing lighting system of the train cabin, allowing the system to coordinate with current light parameters. It may be provided that integration with the existing lighting system refers to the adaptive system working in harmony with the pre-existing lighting setup in the cabin. The effect of this feature is that it enhances the train's current lighting capabilities without requiring a complete overhaul, making it more practical and cost-effective to implement the adaptive lighting system for fatigue management.

In an example, the system operates as an additional lighting apparatus that works in conjunction with the train's existing lighting setup to enhance fatigue management. It may be provided that the additional lighting apparatus supplements the existing system, adding new functionalities such as adaptive control and blue light emission. The effect of this feature is to allow the adaptive system to provide targeted fatigue management while maintaining the general illumination provided by the existing lighting, ensuring both systems complement each other for optimal effectiveness.

In an example, the adaptive lighting system is configured to be retrofit into existing train cabins, allowing for seamless installation without the need for complete lighting system replacement. It may be provided that retrofit refers to the ability to install the new system into older cabins without requiring the removal or replacement of the current lighting infrastructure. The effect of this feature is that it makes the adoption of fatigue management technology more accessible by minimizing the costs and logistical challenges associated with upgrading train cabins.

In an example, the adaptive lighting control system is capable of receiving feedback from environmental sensors to adjust light settings based on time of day, weather conditions, or external light levels. It may be provided that environmental sensors detect external factors that influence lighting needs, such as ambient light levels, weather conditions, or time of day. The effect of this feature is to ensure that the lighting system adapts to the current environment, providing optimal lighting conditions that support fatigue management under various operational circumstances.

In an example, the light-emitting apparatus operates with a duty cycle that provides intervals of blue light emission alternating with periods of warmer light to simulate natural light transitions. It may be provided that a duty cycle refers to the pattern of alternating between blue and warmer light. The effect of this feature is to provide a more natural lighting experience for the operator, helping to regulate circadian rhythms while maintaining the benefits of blue light exposure during key periods of operation.

In an example, the adaptive lighting control system is configured to automatically adjust the wavelength and intensity of light to manage the operator's fatigue during periods of reduced visibility or inclement weather. It may be provided that reduced visibility or inclement weather refers to conditions such as fog, rain, or darkness that impair visual clarity. The effect of this feature is that the system can increase the light output or modify the wavelength to compensate for poor visibility, ensuring that the operator remains alert and safe during challenging operational conditions.

In an example, the train cabin lighting system dynamically personalizes the change of wavelength and intensity of the light-emitting apparatus based on external environmental factors, wherein these factors include one or more of the time of day, duration of use, the operator's level of sleepiness, ambient light intensity, or time of year. It may be provided that the system collects real-time data on these environmental factors to adjust lighting conditions accordingly. An effect of this feature is that the operator experiences tailored lighting that adapts to changing conditions, which helps maintain optimal alertness and performance throughout varying shifts or seasonal changes. By responding to sleepiness or ambient light intensity, the system minimizes fatigue without overwhelming the operator with excessive brightness or color temperature shifts.

In an example, the train cabin lighting system dynamically personalizes the change of wavelength and intensity of the light-emitting apparatus based on the current location of the train and current or predefined safety conditions. It may be provided that the system uses location data and safety protocols to adjust the lighting based on the train's position and its surrounding environment. An effect of this feature is that the operator benefits from lighting that is responsive to both the train's specific operational area and the level of external safety risks. This enhances the operator's situational awareness, ensuring that lighting conditions support a high level of attention during critical safety moments or at specific railway locations that may require heightened vigilance.

In an example, the train cabin lighting system comprises a communication module configured for remote control to perform one or more of software updates, synchronization of personalized settings across multiple devices, and adjusting the controller status across multiple devices. It may be provided that the communication module enables seamless updates and synchronization between devices used by the operator or within the cabin. An effect of this feature is that it allows for efficient management and customization of lighting settings without the need for manual intervention, reducing the operational burden on the operator and ensuring that the system always operates with the most up-to-date configurations. This results in an adaptable lighting system that is easily maintained and responsive to the latest safety and comfort standards.

In an example, the train cabin lighting system integrates an artificial intelligence system utilizing machine learning algorithms to analyze biometric data and historical usage patterns of the operator, dynamically adjusting the wavelength and intensity of the light-emitting apparatus based on the analysis. It may be provided that the Al system continuously learns from the operator's physiological responses and behavioral patterns, allowing the system to fine-tune lighting conditions in real time. An effect of this feature is that it provides a highly personalized lighting experience, improving the operator's alertness and reducing fatigue by adapting to individual needs rather than relying on generic settings. This also enhances the system's ability to optimize energy usage, as it only intensifies or changes lighting when it is truly beneficial for the operator's performance.

In an example, the train cabin lighting system further comprises a sensory feedback mechanism that provides real-time haptic feedback to the operator in response to changing environmental conditions or potential safety hazards. It may be provided that the sensory feedback mechanism delivers subtle vibrations or tactile signals to alert the operator to significant shifts in their surroundings, such as hazardous conditions. An effect of this feature is that it enhances the operator's situational awareness without relying solely on visual or auditory cues, which could be missed or overlooked. By incorporating haptic feedback, the system offers an additional layer of safety, ensuring that the operator is consistently informed about critical events or risks in the railway environment.

In an example, the train cabin lighting system synchronizes the emitted light with ambient lighting conditions, replicating the operator's natural circadian rhythms to promote alertness and overall well-being. It may be provided that sensors in the system monitor external light levels and adjust the interior lighting to mimic natural light cycles. An effect of this feature is that it reduces the operator's fatigue by aligning the lighting with natural biological rhythms, particularly during night shifts or extended hours. This synchronization not only promotes better focus and performance during work but also supports the operator's overall health by preventing disruptions to sleep patterns after their shift.

In an example, the train cabin lighting system comprises a communication module configured to wirelessly communicate with other safety equipment worn by nearby operators, enabling collaborative safety measures and real-time information sharing. It may be provided that this communication module establishes a network between operators, allowing safety information and alerts to be transmitted across multiple devices in real time. An effect of this feature is that it fosters coordinated safety efforts among operators, improving the overall safety of the railway environment by ensuring that all relevant personnel are promptly informed of critical changes or hazards. This collective approach to safety enhances the effectiveness of the lighting system in high-risk or complex railway scenarios.

In an example, the train cabin lighting system comprises a light-emitting apparatus dynamically personalizing the wavelength and intensity based on the current location of the train and based on current or predefined safety conditions. It may be provided that the light-emitting apparatus dynamically adjusts its wavelength and intensity according to the real-time or pre-set safety data, ensuring that the operator's environment is attuned to safety-critical situations. An effect of this feature is that the lighting system can enhance the operator's awareness of surrounding conditions or potential hazards by modulating lighting in response to specific locations or defined risk zones, fostering a controlled alertness adapted to the operational context. The feature provides a location-based feedback mechanism, enabling optimal lighting adjustments with minimal manual input, which not only maintains safety but also allows the system to manage the alertness of the operator effectively across diverse railway zones.

In an example, the train cabin lighting system includes an adaptive lighting control system using real-time location data from a GPS module and predefined hazard zones along the railway to adjust the light wavelength and intensity, alerting the operator to upcoming areas of increased risk by modifying the light output to optimize situational awareness. It may be provided that the adaptive lighting control system receives location data through a GPS module, using this data to reference stored information on hazard zones where elevated attention is required. An effect of this feature is the automated adaptation of the light wavelength and intensity according to upcoming risks, which helps to maintain a heightened level of situational awareness in the operator. By proactively altering light conditions before the train reaches a critical area, the system enables improved reaction times, alertness, and cognitive focus, without requiring additional processing capacity or bandwidth once the data is received, which results in a responsive yet resource-efficient solution for safety monitoring and operator support.

In an example, the train cabin lighting system is configured to control the light-emitting apparatus as signaling means with a warning function to signal hazards in the environment to the operator through specific light signals tailored to the situation. It may be provided that the train cabin lighting system includes a signaling function within the light-emitting apparatus that delivers visual alerts to communicate environmental hazards. An effect of this feature is a reduction in operator dependency on external alert systems, as critical alerts are integrated directly into the cabin lighting, ensuring warnings are immediately visible. The tailored light signals assist the operator in intuitively recognizing varying risk levels, as visual patterns or colors act as distinctive indicators, fostering an additional layer of communication within the cabin that aids in maintaining operational safety and concentration during critical moments without the distraction of audible or external warnings.

In an example, the train cabin lighting system includes a warning function with pre-programmed light patterns or color changes triggered by safety alerts or environmental hazards, such as a flashing blue light for reduced visibility ahead or a gradual color shift to red to indicate proximity to a high-risk zone. It may be provided that the warning function activates pre-set lighting patterns based on the type of safety alert or environmental hazard detected, where each pattern or color change corresponds to a particular hazard level. An effect of this feature is that the operator can interpret alert signals with minimal cognitive load, as the light changes provide an instant and clear indication of the surrounding risk level. By encoding specific hazards into pre-programmed visual patterns, the system achieves an efficient warning mechanism that minimizes delays in situational response and maximizes safety by intuitively conveying risk information through the light-emitting apparatus, supporting sustained focus and reducing the need for additional alert-processing resources.

Any example or embodiment disclosed in relation to any aspect of the present disclosure, is similarly applicable for the other aspects of the present disclosure.

### Brief description of the drawings

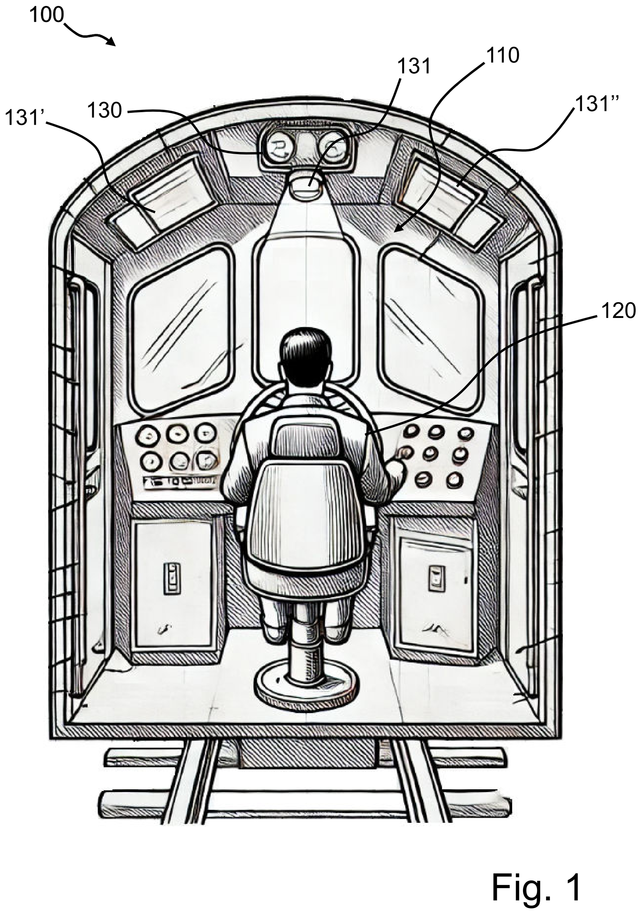

Fig. 1 illustrates a front view of a train cabin lighting system according to an aspect of the present invention.

### Detailed description

Figure 1 depicts an exemplary embodiment of a train cabin lighting system 100 designed to manage the fatigue of a train operator. The figure shows a train cabin interior 110 that accommodates the train operator during operation. The cabin interior 110 includes a seating arrangement, various control elements, and a frontal view for the operator to oversee the train's operation.

Positioned within the cabin is a light-emitting apparatus 130, which comprises at least one light source, however in this example, three light sources 131, 131', 131" are shown, of two different types. These are capable of emitting light across various wavelengths. In practical embodiments, these light sources may include light-emitting diodes (LEDs) that can emit specific wavelengths, particularly blue-enriched light, known to enhance alertness and reduce fatigue during prolonged work hours. The LEDs are capable of emitting light in the blue light range, specifically between 460 nm and 480 nm, as this wavelength has been shown to suppress melatonin production and improve cognitive performance.

The light-emitting apparatus 130 is strategically positioned above the operator's seat to ensure that the emitted light is directed toward the operator's face and workspace. This allows for the management of light intensity and spectral composition, which is critical in maintaining alertness and preventing fatigue-related errors. The system's design ensures that light is diffused across the operator's visual field, avoiding glare or discomfort, which could otherwise impair visual performance. In other embodiments, the light-emitting apparatus 13- may also be even more beneficial be positioned in such a manner that the light is directed onto the retina of the operator.

The system further includes an adaptive lighting control system (not explicitly shown in the figure), which dynamically adjusts one or more of the intensity, spectral composition, and emission angle of the light sources 131, 131', 131" based on real-time feedback. This feedback may come from environmental sensors that detect ambient light conditions outside the cabin or from physiological data gathered from the operator, such as heart rate variability or blink rate. In practical embodiments, this feedback system could incorporate sensors embedded in the operator's seat or wearable devices that monitor fatigue levels.

In this embodiment, the adaptive control system automatically manages the light output to maintain optimal lighting conditions, ensuring that the operator remains alert and productive throughout their shift. The control unit (not shown) is responsible for controlling the light-emitting apparatus 130 based on predefined protocols. These protocols may include a predefined illumination schedule, such as increasing blue light exposure during night shifts and reducing it towards the end of the shift to allow for a smoother transition to rest.

The light-emitting apparatus 130 can also adjust the emission angle or illumination angle based on the operator's position in the cabin. For example, sensors may detect if the operator has adjusted their seating position, and the system will adapt the angle of the light to ensure continued optimal exposure.

This system is designed to work seamlessly with the train's existing lighting infrastructure, either as a retrofit installation or as an additional lighting apparatus that enhances the current system. By incorporating real-time adaptability and environmental feedback, the system provides a customized lighting experience that reduces fatigue and enhances operational safety.

In practice, the train cabin lighting system 100 could be deployed in various types of rail vehicles, ranging from standard locomotives to specialized railway maintenance equipment. The system's design ensures that the operator's performance is optimized through careful management of lighting conditions, improving alertness and reducing the risk of accidents during long or overnight shifts.

## Claims

1. A train cabin lighting system for fatigue management of a train operator, comprising:
a cabin interior configured to accommodate a train operator during operation;
a light-emitting apparatus arranged within the cabin, said apparatus comprising at least one light source capable of emitting light in different wavelengths;
a control unit operatively connected to the light-emitting apparatus, configured to control the emission of light based on predefined protocols;
**characterized by**
the light-emitting apparatus being configured to emit blue light at a wavelength between 460 nm and 480 nm; and
an adaptive lighting control system that dynamically adjusts one or more of the intensity, spectral composition, and emission angle of the light, based on real-time feedback from environmental and operator-specific conditions, to manage the operator's fatigue.

2. The train cabin lighting system according to claim 1, wherein the adaptive lighting control system adjusts the intensity of the light in a range from 50 to 1000 lux, more preferably from 200 to 600 lux, and even more preferably from 300 to 500 lux.

3. The train cabin lighting system according to claim 2, wherein the intensity adjustment is based on real-time feedback from ambient light sensors.

4. The train cabin lighting system according to claim 1, wherein the adaptive lighting control system adjusts the spectral composition of the light between 435 nm and 500 nm, more preferably between 450 nm and 480 nm, and most preferably between 460 nm and 470 nm.

5. The train cabin lighting system according to claim 4, wherein the light-emitting apparatus is configured to emit blue-enriched light with a correlated color temperature (CCT) of 6300K or higher.

6. The train cabin lighting system according to claim 1, wherein the adaptive lighting control system dynamically adjusts the emission angle of the light, more preferably between 20 and 90 degrees, and most preferably between 30 and 60 degrees.

7. The train cabin lighting system according to claim 6, wherein the emission angle is adjusted based on the operator's seating position detected through a sensor.

8. The train cabin lighting system according to claim 1, further comprising a feedback system that monitors operator-specific physiological conditions, including heart rate variability, blink rate, or eye movement, to control the emission of light in real time.

9. The train cabin lighting system according to claim 8, wherein the physiological feedback is used to adjust the duration and intensity of blue light exposure.

10. The train cabin lighting system according to claim 1, wherein the adaptive lighting control system adjusts the duration of blue light emission in cycles, with each cycle lasting between 10 and 60 minutes, more preferably between 15 and 45 minutes, and most preferably between 20 and 30 minutes.

11. The train cabin lighting system according to claim 10, wherein the lighting system provides intermittent blue light exposure interspersed with periods of natural light to minimize circadian disruption.

12. The train cabin lighting system according to claim 1, further comprising a predefined illumination protocol that includes one or more of a predefined duration, pulse shape, duty cycle, intensity, wavelength, or lux of the illuminated light.

13. The train cabin lighting system according to claim 12, wherein the illumination protocol adjusts automatically based on the operator's shift schedule, providing more intense blue light during early night shifts and reducing exposure toward the end of the shift.

14. The train cabin lighting system according to claim 1, wherein the light-emitting apparatus is configured to emit photons at a wavelength between 435 nm and 500 nm, more preferably between 450 nm and 480 nm, and most preferably between 460 nm and 470 nm.

15. The train cabin lighting system according to claim 1, wherein the light-emitting apparatus is arranged for diffusion of light onto a respective eye of the rail worker, ensuring even distribution of light across the operator's visual field.
